# FASCICULE DE BREVET EUROPEEN

(11) **EP 1 027 028 B1**
(45) Date de publication et mention de la délivrance du brevet: **31.07.2002**
(21) Numéro de dépôt: 98947610.6
(22) Date de dépôt: 06.10.1998
(51) Int. Cl.: A61K 7/09

(54) **PROCEDE DE DEFORMATION PERMANENTE DES MATIERES KERATINIQUES SANS RIN AGE INTERMEDIAIRE**
VERFAHREN ZUR DAUERHAFTEN HAARVERFORMUNG KERATINISCHER FASERN OHNE ZWISCHENSPÜLUNG
METHOD FOR PERMANENT SETTING OF KERATIN FIBRES WITHOUT INTERMEDIATE RINSING

(30) Priorité: 10.10.1997 FR 9712713
(43) Date de publication de la demande: 16.08.2000
(73) Titulaire: L'OREAL, 75008 Paris (FR)
(72) Inventeur: NGUYEN, Ly-Lan, F-94240 L'Hay-les-Roses (FR); SABBAGH, Anne, F-92500 Rueil-Malmaison (FR)
(74) Mandataire: Casalonga, Axel
(86) Numéro de dépôt international: FR9802131
(87) Numéro de publication internationale: WO9918922

(56) Documents cités:
- EP-A- 0 065 175
- WO-A-89/07435
- WO-A-96/09030
- FR-A- 2 675 379
- US-A- 5 041 286

## Description

La présente invention est relative à un nouveau procédé de traitement des matières kératiniques et en particulier des cheveux, en vue d'obtenir une déformation permanente de ces dernières.

L'une des techniques couramment utilisée dans le domaine cosmétique pour imprimer aux cheveux une forme durable consiste à procéder à la déformation des cheveux en mettant en oeuvre un agent de réduction puis un agent oxydant.

La technique la plus usuelle pour obtenir une déformation permanente des cheveux consiste, dans un premier temps, à réaliser l'ouverture des liaisons disulfure-S-S de la kératine (cystine) à l'aide d'une composition contenant un agent réducteur, puis après avoir rincé la chevelure ainsi traitée, à reconstituer dans un second temps lesdites liaisons disulfure, en appliquant sur les cheveux préalablement mis sous tension par des bigoudis ou d'autres moyens, ou bien mis en forme ou lissés, une composition oxydante encore appelée "fixateur" de façon à donner finalement aux cheveux la forme recherchée.

Cette technique permet ainsi de réaliser, soit l'ondulation des cheveux, soit leur défrisage ou leur décrêpage ou encore leur lissage.

La nouvelle forme, imposée aux cheveux par un traitement chimique, est durable dans le temps pendant quelques semaines et résiste notamment à l'action des lavages à l'eau ou par des shampoings et ceci par opposition aux techniques mettant en oeuvre des produits de coiffage entraînant une déformation temporaire, telles que de misé en plis, cette déformation disparaissant cependant au coiffage ou aux shampoings.

Les compositions réductrices généralement utilisées pour la première étape d'une opération de permanente, contiennent à titre d'agents réducteurs des sulfites, des bisulfites ou de préférence des thiols.

Dans l'un des modes de déformation permanente des cheveux qui consiste à mettre en forme simplement les cheveux sans les mettre sous tension, notamment avec des compositions épaissies, le rinçage à l'eau après l'étape de réduction conduit à la destruction de la forme donnée initialement, de sorte que les cheveux une fois traités avec la composition fixatrice n'ont pas la forme initialement souhaitée. Par ailleurs, les cheveux traités ont également tendance à perdre de leur couleur naturelle ou de la couleur conférée par une coloration.

Dans FR-A-2 675 379, il est décrit un procédé de mise en forme permanente des cheveux mettant en oeuvré une composition ayant une viscosité et un pouvoir collant prédéterminé, ce procédé étant mis en oeuvre éventuellement sans rinçage des cheveux après l'application de la composition réductrice et avant l'application de la composition oxydante.

Le procédé décrit dans ce document met en oeuvre des compositions réductrices contenant des agents conférant à la composition des propriétés collantes importantes comprises entre 25 et 50 g, qui est la force nécessaire à l'arrachement de deux spatules standardisées entre lesquelles une quaritité précise de la composition réductrice est disposée.

De telles compositions présentent cependant l'inconvénient de mal se rinser et conférer aux cheveux traités et mis en forme des propriétés peu cosmétiques du fait notamment du caractère collant des compositions mises en oeuvre.

La demanderesse a découvert maintenant, ce qui fait l'objet de l'invention, un procédé et des compositions réductrices permettant de mettre en forme les cheveux et n'ayant pas les propriétés collantes définies dans FR-A-2 675 379 et conférant aux cheveux après application de la composition oxydante, et, sans rinçage intermédiaire, des formes permanentes présentant les propriétés cosmétiques souhaitées.

La demanderesse a constaté par ailleurs que le procédé conduisait à un meilleur respect de la couleur naturelle du cheveu ou de celle des cheveux colorés au préalable.

Enfin, le procédé conduit à une économie au niveau des compositions mises en oeuvre.

L'invention a donc pour objet un procédé de déformation permanente des cheveux, comportant l'application d'une composition réductrice épaissie permettant la mise en forme des cheveux et l'application d'une composition fixatrice ou oxydante sans rinçage intermédiaire.

D'autres objets de l'invention apparaîtront à la lecture de la description et des exemples qui suivent.

Le procédé conforme à l'invention est un procédé de mise en forme permanente des fibres kératiniques et en particulier des cheveux, essentiellement caractérisé par le fait :
- qu'on applique sur les fibres kératiniques, de préférence mouillées, une composition réductrice de la kératine, de préférence épaissie, présentant en particulier une viscosité comprise entre 0 à 2500 mPa.s, de préférence entre 100 et 2000 mPa.s, et en particulier entre 200 et 1500 mPa.s au taux d'écoulement laminaire de 100 s⁻¹ et comprise entre 0 à 400 mPa.s au taux d'écoulement laminaire de 600 s⁻¹, cette composition ayant un pouvoir collant inférieure à 15g, la composition réductrice étant appliquée sur les fibres qui sont mis en forme de préférence sans mise sous tension,
- qu'après un temps de pose suffisant pour réduire la kératine, on applique de préférence directement après le temps de pose, une composition oxydante, sans rinçage intermédiaire des fibres,
- qu'on procède au rinçage, de préférence à l'eau, après un temps de pose suffisant pour fixer les fibres mises en forme et réduites lors de la première étape, sous une forme permanente.

La viscosité est mesurée avec un appareil Rheostress RS 50 de Haake. Les mesures ont été effectuées à 25°C, en utilisant le mobile amovible de référence C35/2 vendu avec cet appareil, et en appliquant un taux d'écoulement laminaire de 100 s⁻¹ ou de 600 s⁻¹ pendant 60 secondes.

Les cheveux sont mis en forme par des moyens divers tels que rouleaux, pinces, bandes à crochets, ou simplement à la main du fait de l'utilisation d'une composition épaissie.

Les agents épaississants utilisés dans la composition réductrice sont de préférence choisis parmi des épaississants naturels tels que la gomme de guar, la gomme de Tara, la farine d'épicéa.

Ces épaississants sont utilisés dans la composition réductrice dans des quantités suffisantes pour obtenir les viscosités définies ci-dessus.

La composition est dite non collante si elle ne répond pas au test décrit dans FR-A-2 675 379 mentionné ci-dessus.

Le procédé de mise en forme permanente des cheveux conforme à l'invention est mis en oeuvre de, préférence en procédant à une vaporisation de la composition fixatrice sur les cheveux.

La vaporisation permet d'éviter la destruction de la mise en formé effectuée lors de l'application de la composition réductrice épaissie. Elle permet également un meilleur contrôle de l'application de la composition fixatrice et une imprégnation plus régulière des cheveux.

La composition réductrice de la kératine contient un agent réducteur choisi parmi les sulfites, les bisulfites ou les thiols.

Parmi les composés préférés, on peut citer la cystéine, la cystéamine et leurs dérivés tels que leurs sels cosmétiquement acceptables comme les chlorhydrates, bromhydrates, citrates, acétates, sulfates, l'acide thiolactique, l'acide thioglycolique ainsi que leurs esters, notamment le thioglycolate de glycérol. L'acide thioglycolique est particulièrement efficace et constitue le produit le plus utilisé pour réduire les liaisons disulfure de la kératine.

Dans une forme de réalisation préférée, on utilisera la cystéine et ses sels cosmétiquement acceptables.

Les agents réducteurs sont présents dans des proportions suffisantes pour réduire les liaisons -S-S- de préférence comprises entre 1 et 25%, et en particulier entre 1 et 10% en poids.

Le pH des compositions réductrices est ajusté de façon à avoir un pH compris entré 6,5 et 11,5.

Les agents alcalins sont choisis de préférence parmi la monoéthanolamine, la diéthanolamine, la triéthanolamine, l'isopropylamine, là 2-méthyl 2-amino propanol-1, la propane diamine-1,3, un carbonate ou un bicarbonate alcalin ou d'ammonium, l'ammoniaque, un carbonate organique tel que le carbonate de guanidine ou encore un hydroxyde alcalin, utilisés seuls ou en mélange.

En plus des agents épaississants mentionnés ci-dessus, la composition réductrice peut également contenir d'autres additifs habituellement utilisés dans ces compositions et n'interférant pas avec les propriétés réductrices de la composition.

La composition réductrice peut également contenir des agents tensio-actifs non-ioniques, anioniques, cationiques ou amphotères, couramment utilisés dans de telles compositions. Parmi ceux-ci, on peut citer les alkylsulfates, les alkylbenzènesulfates, les alkyléthersulfates, les alkylsulfonates, les sels d'ammonium quaternaire, les alkylbétaïnes, les alkylphénols oxyéthylénés, les alcanolamides d'acides gras, les esters d'acides gras oxyéthylénés, ainsi que des tensio-actifs non-ioniques de la famille des hydroxypropyléthers.

Ces agents tensio-actifs sont généralement utilisés dans des proportions maximales de 30%, et de préférence comprises entre 0,5 et 10% en poids par rapport au poids total de la composition.

Ces compositions peuvent également contenir des agents traitants tels que des silicones volatiles ou non, linéaires ou cycliques ou leurs mélanges. Parmi les silicones, on peut citer les polydiméthylsiloxanes, les polyorganosiloxanes quaternisés tels que décrits dans FR-A-2 535 730, les polyorganosiloxanes à groupement aminoalkyle modifiés par des groupements alcoxycarbonylalkyle tels que décrits dans le brevet US-A-4 749 732, les polyorganosiloxanes tels que les copolymères de polydiméthylsiloxane-polyoxyalkyle tels que le diméthicone copolyol, un polydiméthylsiloxane à groupements terminaux stéaroxy-(stéaroxydiméthicone), un copolymère polydiméthylsiloxane dialkylammonium acétate ou un copolymère polydiméthylsiloxane polyalkylbétaïne décrit dans GB-A-2 197 352, des polysiloxanes organomodifiés par des groupements mercapto ou mercapto-alkyle tels que décrits dans FR-B-1 530 369 et EP-A-0 295 780, ainsi que des silanes tels que le stéaroxy-triméthylsilane.

D'autres ingrédients utilisables dans les compositions réductrices conformes à l'invention sont choisis parmi les cires, les polymères choisis parmi les polymères cosmétiquement acceptables anioniques, cationiques, non-ioniques ou amphotères, les agents de gonflement et de pénétration permettant de renforcer l'efficacité du réducteur tels que le diméthylisosorbitol, l'urée et ses dérivés, la pyrrolidone, les n-alkylpyrrolidone, la thiamorpholinone, les alkyléthers d'alkylèneglycol ou de dialkylèneglycol tels que par exemple le monométhyléther de propylèneglycol, le monométhyléther de dipropylèneglycol, des alcanediols en C₃-C₆ tels que le propanediol-1,2, l'imidazolidinone-2, ainsi que d'autres composés tels que des alcools gras, des dérivés de lanoline, des céramides, notamment les céramides elles-mêmes , les glycocéramides, les pseudocéramides décrits notamment dans FR-A-95 12399, et dans DOWNING Journal of Lipid Research, Vol. 35, p. 2060, 1994, ou dans FR-A-2 673 179, EP-A-0227994, WO-94/07844, WO-92/05764, des ingrédients actifs tels que l'acide pantothénique, des agents anti-chute, des agents anti-pelliculaires, des agents de mise en suspension, des agents séquestrants, des agents opacifiants, des colorants, des filtres solaires, ainsi que des parfums et des conservateurs.

La composition oxydante, appliquée de préférence par vaporisation sur les cheveux imprégnés de la composition réductrice et mis en forme, est une composition aqueuse contenant un agent oxydant choisi parmi l'eau oxygénée, les peroxydes d'urée, les bromates tels que les bromates alcalins, les persels ou un mélange de bromates alcalins et d'un persel.

Selon un mode de réalisation préféré, l'agent oxydant est constitué par l'eau oxygénée présent dans des proportions comprises entre 1 et 10 volumes, et de préférence de l'ordre de 8 volumes.

Lorsque les bromates sont utilisés, la concentration en bromates alcalins est de 1 à 12% et celle en persels de 0,1 à 15% en poids par rapport au poids total de la composition oxydante.

Le pH de ces compositions est généralement acide et il est compris habituellement entre 2 et 9, et de préférence entre 3 et 8.

Lorsque l'eau oxygénée est utilisée, elle peut être stabilisée par la phénacétine, l'acétaniline, les phosphates mono- et trisodiques ou par les sulfates d'hydroxy-8 quinoléine.

On procède à l'application de la composition oxydante, de préférence par vaporisation, après un temps de pose suffisant pour réduire les liaisons -S-S-, généralement compris entre 2 et 30 minutes, et de préférence entre 5 et 20 minutes. On peut utiliser pendant cette phase d'attente un bonnet ou un gel de protection.

La composition oxydante est maintenue au contact des cheveux pendant une durée suffisante pour fixer la déformation permanente qui est de l'ordre de 5 à 30 minutes et en particulier entre 5 et 15 minutes, puis on procède à un rinçage abondant à l'eau des cheveux ainsi traités, au séchage et au coiffage pour la mise en forme permanente.

Les exemples suivants sont destinés à illustrer l'invention sans pour autant présenter un caractère limitatif.

### EXEMPLES

### EXEMPLE 1

### Réducteur

- Acide thioglycolique 5 g
- Acide diéthylène triamine pentacétique, sel pentasodique en solution aqueuse à 40% 0,2 g
- Ammoniaque à 20% qs pH 7,9
- Carbonate d'ammonium 5 g
- Parfum 0,5 g
- Alcool oléique oxyéthyléné (20 moles d'oxyde d'éthylène) 1 g
- Eau déminéralisée qs 100 g

### Fixateur

- Eau oxygénée à 50% 4,8 g
- Stabilisants 0,2 g
- Acide citrique qs pH 3
- Eau déminéralisée qs 100 g

On applique le réducteur liquide non collant sur les cheveux lavés et essorés. On met en forme les cheveux, par exemple, en formant des boucles maintenues par des pinces. On laisse poser 15 minutes, puis on vaporise uniformément le fixateur. On laisse poser 5 minutes. On rince alors les cheveux.

On obtient ainsi, simplement et rapidement, une déformation durable de la fibre qui permet d'avoir un recoiffage plus facile dans le temps.

### EXEMPLE 2

### Réducteur

- Cystéine 3 g
- Gomme de guar 1 g
- Farine d'épicéa 8 g
- Monoéthanolamine 2,2 g
- Parfum 0,5 g
- Alcool oléique oxyéthyléné (20 moles d'oxyde d'éthylène) 1 g
- Mélange cocoyl amidopropyl bétaïne/monolaurate de glycérol en solution aqueuse à 30% 1,8 g
- Acide diéthylène triamine pentacétique, sel pentasodique en solution aqueuse à 40% 0,2 g
- Homopolymère chlorure de diméthyl diallyl ammonium en solution aqueuse à 40% 2,5 g
- Eau déminéralisée qs 100 g

### Fixateur

- Bromate de sodium 7,5 g
- Tampon phosphate pH 7,5
- Eau déminéralisée qs 100 g

Le réducteur faiblement épaissi (1400 mPa.s à 100s⁻¹ et 200 à 600s⁻¹), de collant égal à 10mg, de pH 9,1, permet de mettre en forme les cheveux dans la position souhaitée sans l'aide-de matériel de mise sous tension.

Ce produit est appliqué sur les cheveux lavés et essorés, puis on met en forme les cheveux. On laisse poser 15 minutes, puis on vaporise le fixateur. On laisse poser 10 minutes, puis on rince. L'élimination au rinçage des produits est facile.

On obtient une déformation durable des cheveux qui permet un coiffage plus facile dans le temps.

## Revendications

1. Procédé de mise en forme permanente des fibres kératiniques et en particulier des cheveux, **caractérisé par le fait que** :
- l'on applique sur les fibres kératiniques une composition réductrice de la kératine, ayant un pouvoir collant inférieur à 15g et une viscosité de 0 à 2500 mPa.s aux taux d'écoulement laminaire de 100s⁻¹ et on les met en forme,
- après un temps de pose suffisant pour réduire la kératine, on applique une composition oxydante, sans rinçage intermédiaire des fibres,
- on procède au rinçage après un temps suffisant pour fixer les fibres mises en forme et réduites sous une forme permanente.

2. Procédé selon la revendication 1, **caractérisé par le fait que** la viscosité de la composition réductrice est comprise entre 100 et 2000 mPa.s au taux d'écoulement laminaire de 100 s⁻¹.

3. Procédé selon l'une quelconque des revendications 1 à 2, **caractérisé par le fait que** la viscosité est comprise entre 200 et 1500 mPa.s au taux d'écoulement laminaire de 100 s⁻¹.

4. Procédé selon l'une quelconque des revendications 1 à 3, **caractérisé par le fait que** la composition réductrice a une viscosité comprise entre 0 et 400 mPa.s au taux d'écoulement laminaire de 600 s⁻¹.

5. Procédé selon l'une quelconque des revendications 1 à 4, **caractérisé par le fait que** la composition est épaissie avec des épaississants naturels choisis parmi la gomme de guar, la gomme de Tara, la farine d'épicéa.

6. Procédé selon l'une quelconque des revendications 1 à 5, **caractérisé par le fait qu'**elle a un pouvoir collant inférieur à 15 g, correspondant à la force nécessaire à l'arrachement de 2 spatules standardisées, entre lesquelles une quantité déterminée de la composition réductrice a été déposée.

7. Procédé selon l'une quelconque des revendications 1 à 6, **caractérisé par le fait que** la composition oxydante ou fixatrice est appliquée sur les fibres mises en forme avec la composition réductrice épaissie par vaporisation.

8. Procédé selon l'une quelconque des revendications 1 à 7, **caractérisé par le fait que** la composition réductrice de la kératine contient un réducteur choisi parmi les sulfites, les bisulfites et les thiols.

9. Procédé selon l'une quelconque des revendications 1 à 8, **caractérisé par le fait que** les agents réducteurs de la kératine sont choisis parmi la cystéine, la cystéamine et leurs sels cosmétiquement acceptables, l'acide thiolactique, l'acide thioglycolique et leurs esters cosmétiquement acceptables.

10. Procédé selon l'une quelconque des revendications 1 à 9, **caractérisé par le fait que** les agents réducteurs de la kératine sont présents dans des concentrations comprises entre 1 et 25% en poids et de préférence entre 1 et 10% en poids.

11. Procédé selon l'une quelconque des revendications 1 à 10, **caractérisée par le fait que** la composition réductrice a un pH compris entre 6,5 et 11,5.

12. Procédé selon l'une quelconque des revendications 1 à 11, **caractérisée par le fait que** le pH est ajusté par des agents absorbés choisis de préférence parmi la la monoéthanolamine, la diéthanolamine, la triéthanolamine, l'isopropylamine, la 2-méthyl 2-amino propanol-1, la propane diamine-1,3, un carbonate ou un bicarbonate alcalin ou d'ammonium, l'ammoniaque, un carbonate organique tel que le carbonate de guanidine ou encore un hydroxyde alcalin, utilisés seuls ou en mélange.

13. Procédé selon l'une quelconque des revendications 1 à 12, **caractérisé par le fait que** la composition réductrice contient également des agents tensio-actifs non-ioniques, anioniques, cationiques ou amphotères.

14. Procédé selon l'une quelconque des revendications 1 à 13, **caractérisé par le fait que** la composition réductrice contient des agents traitants choisis parmi les silicones volatiles ou non, linéaires ou cycliques, ou leurs mélanges, les cires, les polymères, des agents de pénétration, des agents de gonflement, des alkyléthers d'alkylèneglycol ou de dialkylèneglycol, des alcanediols, des alcools gras, des dérivés de lanoline, des céramides, des ingrédients actifs, des agents anti-chute, anti-pelliculaires, des agents de mise en suspension, des agents séquestrants, des agents opacifiants, des colorants, des filtres solaires, des parfums, des conservateurs ou leurs mélanges.

15. Procédé selon l'une quelconque des revendications 1 à 14, **caractérisé par le fait que** la composition oxydante ou fixatrice est appliquée par vaporisation directement et sans rinçage intermédiaire sur les fibres kératiniques mises en forme et réduites par la composition réductrice, après un temps de pose de la composition réductrice compris entre 2 et 30 minutes.

16. Procédé selon l'une quelconque des revendications 1 à 15, **caractérisé par le fait que** l'oxydant de la composition fixatrice est choisi parmi lc peroxyde d'hydrogène, le peroxyde d'urée, les bromates, les persels et leurs mélanges.

17. Procédé selon l'une quelconque des revendications 1 à 16, **caractérisé par le fait que** la composition oxydante est maintenue au contact des fibres pendant une durée de 5 à 30 minutes.

## Patentansprüche

1. Verfahren zur permanenten Verformung von Keratinfasern und insbesondere des Haares, **dadurch gekennzeichnet, daß**:
- eine auf das Keratin reduzierend wirkende Zusammensetzung mit einer Klebrigkeit unter 15 g und einer Viskosität bei einer laminaren Strömungsgeschwindigkeit von 100 s⁻¹ im Bereich von 0 bis 2500 mPa·s auf die Keratinfasern aufgetragen wird und die Keratinfasern in Form gebracht werden;
- nach einer Einwirkzeit, die ausreichend ist, um das Keratin zu reduzieren, eine oxidierende Zusammensetzung aufgetragen wird, ohne daß die Fasern zwischenzeitlich gespült werden; und
- nach einer Zeitspanne, die ausreichend ist, um die in Form gebrachten und reduzierten Fasern in einer dauerhaften Form zu fixieren, die Fasern gespült werden.

2. Verfahren nach Anspruch 1, **dadurch gekennzeichnet, daß** die Viskosität der reduzierenden Zusammensetzung bei einer laminaren Strömungsgeschwindigkeit von 100 s⁻¹ im Bereich von 100 bis 2000 mPa·s liegt.

3. Verfahren nach einem der Ansprüche 1 bis 2, **dadurch gekennzeichnet, daß** die Viskosität bei einer laminaren Strömungsgeschwindigkeit von 100 s⁻¹ im Bereich von 200 bis 1500 mPa·s liegt.

4. Verfahren nach einem der Ansprüche 1 bis 3, **dadurch gekennzeichnet, daß** die reduzierende Zusammensetzung bei einer laminaren Strömungsgeschwindigkeit von 600 s⁻¹ eine Viskosität im Bereich von 0 bis 400 mPa·s hat.

5. Verfahren nach einem der Ansprüche 1 bis 4, **dadurch gekennzeichnet, daß** die Zusammensetzung mit natürlichen Verdikkungsmitteln verdickt wird, die unter Guargummi, Tara-Kernmehl und Fichtenmehl ausgewählt sind.

6. Verfahren nach einem der Ansprüche 1 bis 5, **dadurch gekennzeichnet, daß** sie eine Klebrigkeit unter 15 g aufweist, die der Kraft entspricht, die erforderlich ist, um zwei genormte Spatel zu trennen, zwischen die eine vorbestimmte Menge der reduzierenden Zusammensetzung gebracht wurde.

7. Verfahren nach einem der Ansprüche 1 bis 6, **dadurch gekennzeichnet, daß** die oxidierende oder fixierende Zusammensetzung durch Sprühen auf die mit der dickflüssigen, reduzierenden Zusammensetzung in Form gebrachten Fasern aufgebracht wird.

8. Verfahren nach einem der Ansprüche 1 bis 7, **dadurch gekennzeichnet, daß** die auf das Keratin reduzierend wirkende Zusammensetzung ein Reduktionsmittel enthält, das unter den Sulfiten, Bisulfiten und Thiolen ausgewählt ist.

9. Verfahren nach einem der Ansprüche 1 bis 8, **dadurch gekennzeichnet, daß** die auf das Keratin reduzierend wirkenden Mittel unter Cystein, Cysteamin und deren kosmetisch akzeptablen Salzen, Thiomilchsäure, Thioglykolsäure und deren kosmetisch akzeptablen Estern ausgewählt sind.

10. Verfahren nach einem der Ansprüche 1 bis 9, **dadurch gekennzeichnet, daß** die Reduktionsmittel für das Keratin in Konzentrationen im Bereich von 1 bis 25 Gew.-% und vorzugsweise 1 bis 10 Gew.-% vorliegen.

11. Verfahren nach einem der Ansprüche 1 bis 10, **dadurch gekennzeichnet, daß** die reduzierende Zusammensetzung einen pH-Wert im Bereich von 6,5 bis 11,5 aufweist.

12. Verfahren nach einem der Ansprüche 1 bis 11, **dadurch gekennzeichnet, daß** der pH-Wert mit Mitteln eingestellt wird, die vorzugsweise unter Monoethanolamin, Diethanolamin, Triethanolamin, Isopropylamin, 2-Methyl-2-amino-1-propanol, 1,3-Diaminopropan, Alkalicarbonaten, Alkalibicarbonaten, Ammoniumcarbonat, Ammoniumbicarbonat, Ammoniak, organischen Carbonaten, wie Guanidincarbonat, oder Alkalihydroxiden ausgewählt sind, wobei diese Verbindungen einzeln oder im Gemisch verwendet werden.

13. Verfahren nach einem der Ansprüche 1 bis 12, **dadurch gekennzeichnet, daß** die reduzierende Zusammensetzung auch nichtionische, anionische, kationische oder amphotere grenzflächenaktive Stoffe enthält.

14. Verfahren nach einem der Ansprüche 1 bis 13, **dadurch gekennzeichnet, daß** die reduzierende Zusammensetzung Behandlungsmittel enthält, die unter den flüchtigen oder nicht flüchtigen, geradkettigen oder cyclischen Siliconen oder deren Gemischen, Wachsen, Polymeren, Penetrationsmitteln, Quellmitteln, Alkylenglykolalkylethern, Dialkylenglykolalkylethern, Alkandiolen, Fettalkoholen, Lanolinderivaten, Ceramiden, Wirkstoffen, Wirkstoffen gegen Haarausfall, Wirkstoffen gegen Schuppen, Suspendiermitteln, Maskierungsmitteln, Trübungsmitteln, Farbmitteln, Sonnenschutzfiltern, Parfums, Konservierungsmitteln oder deren Gemischen ausgewählt sind.

15. Verfahren nach einem der Ansprüche 1 bis 14, **dadurch gekennzeichnet, daß** die oxidierende oder fixierende Zusammensetzung auf die durch die reduzierende Zusammensetzung in Form gebrachten und reduzierten Keratinfasern nach einer Einwirkzeit der reduzierenden Zusammensetzung von 2 bis 30 min durch Sprühen direkt und ohne zwischenzeitlichen Spülschritt aufgebracht wird.

16. Verfahren nach einem der Ansprüche 1 bis 15, **dadurch gekennzeichnet, daß** das Oxidationsmittel der fixierenden Zusammensetzung unter Wasserstoffperoxid, Harnstoffperoxid, Bromaten, Salzen von Persäuren und deren Gemischen ausgewählt ist.

17. Verfahren nach einem der Ansprüche 1 bis 16, **dadurch gekennzeichnet, daß** die oxidierende Zusammensetzung während einer Zeitspanne von 5 bis 30 min mit den Fasern in Kontakt belassen wird.

## Claims

1. Process for permanently shaping keratin fibres, and in particular the hair, **characterized in that**:
- a keratin-reducing composition is applied to the keratin fibres, this composition having a bonding power of less than 15 g and a viscosity of from 0 to 2500 mPa.s at a laminar flow rate of 100 s⁻¹, and the fibres are placed in shape,
- after an exposure time which is sufficient to reduce the keratin, an oxidizing composition is applied, without intermediate rinsing of the fibres,
- after a time which is sufficient to fix the fibres placed in shape and reduced, in a permanent shape, rinsing is carried out.

2. Process according to Claim 1, **characterized in that** the viscosity of the reducing composition is between 100 and 2000 mPa.s at a laminar flow rate of 100 s⁻¹.

3. Process according to any one of Claims 1 to 2, **characterized in that** the viscosity is between 200 and 1500 mPa.s at a laminar flow rate of 100 s⁻¹.

4. Process according to any one of Claims 1 to 3, **characterized in that** the reducing composition has a viscosity of between 0 and 400 mPa.s at a laminar flow rate of 600 s⁻¹.

5. Process according to any one of Claims 1 to 4, **characterized in that** the composition is thickened with natural thickeners chosen from guar gum, tara gum and spruce meal.

6. Process according to any one of Claims 1 to 5, **characterized in that** it has a bonding power of less than 15 g, corresponding to the force required to pull apart 2 standardized spatulas between which a determined amount of the reducing composition has been applied.

7. Process according to any one of Claims 1 to 6, **characterized in that** the oxidizing or fixing composition is applied to the fibres, placed in shape with the thickened reducing composition, by vaporization.

8. Process according to any one of Claims 1 to 7, **characterized in that** the keratin-reducing composition contains a reducing agent chosen from sulphites, bisulphites and thiols.

9. Process according to any one of Claims 1 to 8, **characterized in that** the keratin-reducing agents are chosen from cysteine, cysteamine and their cosmetically acceptable salts, thiolactic acid, thioglycolic acid and their cosmetically acceptable esters.

10. Process according to any one of Claims 1 to 9, **characterized in that** the keratin-reducing agents are present in concentrations of between 1 and 25% by weight and preferably between 1 and 10% by weight.

11. Process according to any one of Claims 1 to 10, **characterized in that** the reducing composition has a pH of between 6.5 and 11.5.

12. Process according to any one of Claims 1 to 11, **characterized in that** the pH is adjusted using absorbed agents preferably chosen from monoethanolamine, diethanolamine, triethanolamine, isopropylamine, 2-methyl-2-amino-1-propanol, propane-1,3-diamine, an ammonium or alkali metal carbonate or bicarbonate, aqueous ammonia, an organic carbonate such as guanidine carbonate, or an alkali metal hydroxide, which are used alone or as a mixture.

13. Process according to any one of Claims 1 to 12, **characterized in that** the reducing composition also contains nonionic, anionic, cationic or amphoteric surfactants.

14. Process according to any one of Claims 1 to 13, **characterized in that** the reducing composition contains treating agents chosen from volatile or non-volatile, linear or cyclic silicones or mixtures thereof, waxes, polymers, penetrating agents, swelling agents, alkyl ethers of alkylene glycol or of dialkylene glycol, alkanediols, fatty alcohols, lanolin derivatives, ceramides, active ingredients, agents for preventing hair loss, antidandruff agents, suspending agents, sequestering agents, opacifiers, colorants, sunscreens, fragrances and preserving agents, or mixtures thereof.

15. Process according to any one of Claims 1 to 14, **characterized in that** the oxidizing or fixing composition is applied by vaporizing it, directly and without intermediate rinsing, onto the keratin fibres which have been placed in shape and reduced by the reducing composition, after an exposure time of the reducing composition of between 2 and 30 minutes.

16. Process according to any one of Claims 1 to 15, **characterized in that** the oxidizing agent in the fixing composition is chosen from hydrogen peroxide, urea peroxide, bromates, persalts and mixtures thereof.

17. Process according to any one of Claims 1 to 16, **characterized in that** the oxidizing composition is kept in contact with the fibres for a period of from 5 to 30 minutes.
